# EUROPEAN PATENT SPECIFICATION

(11) **EP 1 845 167 B1**
(45) Date of publication and mention of the grant of the patent: **13.11.2013**
(21) Application number: 07007289.7
(22) Date of filing: 07.04.2007
(51) Int. Cl.: C12Q 1/68

(54) **Method for quick determination of cytokeratin 19 (CK19) and primers and probes therefore**
Verfahren zur schnellen Bestimmung von Cytokeratin 19 (CK19) und Primer und Sonden dafür
Procédé de détermination rapide de cytokératine 19 (CK19) et les amorces et les sondes correspondantes

(30) Priority: 11.04.2006 JP 2006108744
(43) Date of publication of application: 17.10.2007
(73) Proprietor: Roche Diagnostics GmbH, 68305 Mannheim (DE); F.Hoffmann-La Roche AG, 4070 Basel (CH)
(72) Inventor: Kitagawa, Yuko, Tokyo 156-0051 (JP); Sakakura, Yasuhiko, Yamato City Kanagawa Pref. 242-0024 (JP)

(56) References cited:
- EP-A- 1 510 587
- WO-A-96/17080
- WO-A-03/089899
- WO-A-2005/118875
- WO-A1-2007/020081
- US-A1- 2006 068 418
- VAN TRAPPEN P O ET AL: "Molecular quantification and mapping of lymph-node micrometastases in cervical cancer" LANCET THE, LANCET LIMITED. LONDON, GB, vol. 357, no. 9249, 6 January 2001 (2001-01-06), pages 15-20, XP004799755 ISSN: 0140-6736
- BJARNADOTTIR HELGA ET AL: "A rapid real-time qRT-PCR assay for ovine beta-actin mRNA" JOURNAL OF BIOTECHNOLOGY, vol. 117, no. 2, May 2005 (2005-05), pages 173-182, XP002437945 ISSN: 0168-1656
- AERTS J ET AL: "A real-time quantitative reverse transcriptase polymerase chain reaction (RT-PCR) to detect breast carcinoma cells in peripheral blood" ANNALS OF ONCOLOGY, KLUWER, DORDRECHT, NL, vol. 12, no. 1, January 2001 (2001-01), pages 39-46, XP009074839 ISSN: 0923-7534
- SAVTCHENKO E S ET AL: "EMBRYONIC EXPRESSION OF THE HUMAN 40-KD KERATIN EVIDENCE FROM A PROCESSED PSEUDOGENE SEQUENCE" AMERICAN JOURNAL OF HUMAN GENETICS, vol. 43, no. 5, 1988, pages 630-637, XP002437942 ISSN: 0002-9297
- RUUD P ET AL: "Identification of a novel cytokeratin 19 pseudogene that may interfere with reverse transcriptase-polymerase chain reaction assays used to detect micrometastatic tumor cells" INTERNATIONAL JOURNAL OF CANCER, NEW YORK, NY, US, vol. 80, 1999, pages 119-125, XP002984313 ISSN: 0020-7136
- WHITTOCK N V ET AL: "Genomic organization and amplification of the human keratin 15 and keratin 19 genes" BIOCHEMICAL AND BIOPHYSICAL RESEARCH COMMUNICATIONS, ACADEMIC PRESS INC. ORLANDO, FL, US, vol. 267, 2000, pages 462-465, XP002972142 ISSN: 0006-291X
- MUTIMER H ET AL: "PITFALLS OF PROCESSED PSEUDOGENES IN RT-PCR" BIOTECHNIQUES, INFORMA LIFE SCIENCES PUBLISHING, WESTBOROUGH, MA, US, vol. 24, no. 4, April 1998 (1998-04), pages 585-588, XP008050069 ISSN: 0736-6205
- RAFF T ET AL: "DESIGN AND TESTING OF BETA-ACTIN PRIMERS FOR RT-PCR THAT DO NOT CO-AMPLIFY PROCESSED PSEUDOGENES" BIOTECHNIQUES, INFORMA LIFE SCIENCES PUBLISHING, WESTBOROUGH, MA, US, vol. 23, no. 3, 1997, pages 456-458,460, XP001206667 ISSN: 0736-6205
- DIMMLER ARNO ET AL: "Transcription of cytokeratins 8, 18, and 19 in bone marrow and limited expression of cytokeratins 7 and 20 by carcinoma cells: Inherent limitations for RT-PCR in the detection of isolated tumor cells" LABORATORY INVESTIGATION, vol. 81, no. 10, October 2001 (2001-10), pages 1351-1361, XP002448334 ISSN: 0023-6837

## Description

### Field of the invention

The present invention relates to methods for the determination of CK19 mRNA, and for the determination of CK19 and CK20 mRNA, particularly in a clinical setting. Furthermore, probes and PCR primers as well as kits used for quick and specific amplification and detection of mRNA of CK19 on a real-time basis are provided.

### Background of the invention

Cytokeratin is a group of proteins called an intermediate-sized filament forming cytoskeleton and, up to now, not less than 20 kinds of subspecies have been reported. Those cytokeratins are mostly present in epithelial cells and distribution of each subspecies is different depending upon the type of epithelial cells.

Cytokeratin 19 (CK19) has been reported to be expressed more abundantly in cancer tissues such as breast cancer, stomach cancer, prostate cancer and lung cancer than in normal tissues and has been investigated for its application to clinical diagnosis as a clinical marker for diagnosis of cancer and also as an index for selecting a therapeutic strategy. For example, in breast cancer in early stage or in stomach cancer in early stage, there is a possibility that the appropriateness for reducing the range for lymph node dissection during a surgical operation for improvement of quality of life (QOL) of a patient may be judged by diagnosis for metastasis of cancer in lymph node using CK19 as an index. There may be also a possibility that, when the presence or absence of cancer cells in a washing of peritoneum is checked using CK19 as an index, the risk of a peritoneal seedling of a patient suffering from progressive stomach cancer may be evaluated.

With regard to a method for detection a gene in high sensitivity a LAMP method, a TRC method, a PCR method, etc. have been reported up to now.

A LAMP (loop mediated isothermal amplification) method is an art where an aimed gene is amplified efficiently at predetermined temperature using four kinds of primers and DNA polymerase of a chain substitution type which are designed to any six regions in gene regions to be amplified (WO 00/28082).

A TRC (transcription reverse transcription concerted reaction) method is an art where RNA is amplified at a predetermined temperature (EP 0969101) where an aimed RNA region is amplified by means of trimming of a target RNA using an RNase activity of a reverse transcriptase and DNA oligonucleotide for cleaving of target RNA called a scissor probe and by means of repetition of reverse transcription reaction and transcription reaction. In this art, RNA is able to be directly amplified and, therefore, a reverse transcription step which is necessary in PCR and LAMP method is not necessary.

Steps for amplification are complicated in those amplifying methods for nucleic acid as compared with a PCR method and, in addition, it is not possible to amplify plural targets at the same time and to detect each of them in one reaction tube. This means that an internal control for discrimination of false negative and real negative can not be integrated in a reaction system. Accordingly, such methods have the problem that, when applied to clinical diagnosis, false negative samples may not be discriminated, etc.

A PCR method is an art where any DNA region may be amplified efficiently with good reproducibility and, at present, it has been widely used throughout the world from fundamental studies to practical industries in the fields of medicine, medical jurisprudence, veterinary science, agricultural science, pharmaceutical science, biology, etc. When this art is combined with an enzyme having a reverse transcription activity, it is also possible to amplify any mRNA region (RT-PCR). Many means for detection of amplified products have been reported as well. When a fluorescent probe where a fluorescent dye is bonded to a DNA oligonucleotide is added to a PCR solution, monitoring of the amplifying reaction may be carried out on a real-time basis (real-time PCR). It is also possible that, when a fluorescent probe where plural fluorescent dyes are separately labeled is used, plural target DNAs (or RNAs) are amplified at the same time in a reactor and the mode of such an amplifying reaction may also be separately monitored (multiplex real-time PCR).

Although amplification of mRNA by an RT-PCR method is highly sensitive, the gene (DNA) which is a plan for the mRNA is also amplified in case human DNA is present in the sample to be tested, even if present only in a small amount. Accordingly, it may be difficult to judge whether the amplified product is derived from RNA or DNA.

In such methods there is a certain possibility for "false positive" results, in which the result which is to be negative is judged to be positive. This is a big problem when such methods are applied to clinical diagnosis.

As a means for avoiding such a problem, a design is carried out in amplification of RNA by an RT-PCR so that an intron comes into the region of the gene corresponding to such an RNA region.

In general, a gene is divided by an intron, which is a region having no genetic information. Accordingly, if an intron is present in a region which is amplified by RT-PCR, the PCR product where DNA was amplified is larger to the extent of the intron compared to the amplified product of mRNA. In that case, even when DNA is contaminating an mRNA sample to be subjected to PCR, it is possible to judge whether mRNA is amplified or DNA is amplified by means of an agarose gel electrophoresis or the like where the difference in size of the amplified product may be detected.

However, when a gene region where no intron is present is amplified, the size of the amplified product entirely is the same as that in case of an mRNA. In this case it is not possible to judge by electrophoresis.

When a human gene (DNA) is contaminating a clinical sample, there is another problem which is the presence of a process-type pseudogene. A pseudogene does not function as a gene and, since no intron is present in a base sequence of a process-type pseudogene and since there is a high homology in view of base sequence to mRNA, it is not possible to judge by way of the size of the amplified product.

The presence of process-type pseudogene in CK19 has been reported. The homology of the pseudogene to CK19 mRNA is not less than 90%. Accordingly, if amplification of the pseudogene takes place, there is a possibility that it is not possible to judge whether the amplified product is derived from mRNA or derived from the pseudogene comparable to the case of PCR of a gene region having no intron.

Thus, in case where CK19 mRNA is detected by a nucleic acid amplification method such as RT-PCR to be used in a clinical test, DNA which is contaminating the sample should be thoroughly excluded. In other words, when the resulting positive result is interpreted, its application to a clinical test is not possible unless it may be discriminated whether the positive result is "true positive" due to CK19 mRNA or is "false positive" due to CK19 gene or CK19 pseudogene.

An effective means for preventing the generation of such a "false positive" is that human DNA in the sample is thoroughly excluded. A method for preparing a sample for such a purpose has already been reported. However, in the method for preparing a sample as such, too much attention is paid on the degree of purification and, as a result, the time necessary for preparing the sample is long, a special apparatus is necessary and numbers of the samples which may be treated are limited. Therefore, the method is not always effective in the clinical field where quickness and simplicity are demanded.

There have been many publications reporting PCR primers and detection probes for amplification and detection of CK19 mRNA by means of RT-PCR and there also have been reports mentioning that a special device is used not to amplify or detect the CK19 pseudogene (Yuan, CC., et al., Gynecol. Oncol., 85: (1) 148-153, 2002; Dimmler, A. et al., Laboratory Investigation, 81: (10) 1351-1361, 2000; Gazzaniga, P., et al., Clin. Cancer Res., 7: 577-583, 2001; Stathopoulou, A., et al., Clin. Cancer Res., 9: 5145-5151, 2003; Fellowes, V., et al., Int. J. Oncol., 24: 861-867, 2004). However, none of these publications does satisfy the quickness which is demanded in the clinical field at present.

For example, a nested PCR is conducted in Yuan et al.. However, in this method a time of 5 hours or longer is required in the PCR only. Particularly in Yuan et al. and in Stathopoulou et al. an agarose gel electrophoresis is used for detection of the amplified product whereby the judgment of the result of the PCR requires longer time. In addition, in preparing the sample for the PCR, all of these prior art documents (Yuan, CC., et al.; Dimmler, A. et al.; Gazzaniga, P., et al.; Stathopoulou, A., et al.; Fellowes, V.) carry out the PCR and the reverse transcription reaction of mRNA in separate reactions (two-step RT-PCR) and about one hour is necessary for the reverse transcription step only.

In Dimmler et al. and Fellowes et al. it is mentioned that the purified RNA sample is to be further subjected to a DNase treatment. In the practical medical field where a nucleic acid amplification method is used for clinical diagnosis, pollution with nuclease is not preferred. That is because, if pollution by DNase or RNase occurs in an operation chamber or in experimental instruments, there is a possibility of affecting the result of the gene test. Accordingly, with regard to a method for preparing a sample for nucleic acid amplification to be used in a clinical test, a method where no nuclease is used usually is preferred.

WO 2005118875 A discloses a method for intraoperative diagnosis of breast cancer marker CK19 mRNA by quantitative RT-PCR with primers and TaqMan probe spanning an exon-exon junction.

### Brief Description of the Drawings

Fig. 1 is a graph which shows the result where CK19 mRNA which was diluted in a stepwise manner was subjected to the one-step RT-PCR of the present invention.
Fig. 2 is a graph which shows the result where human DNA and CK19 pseudogene were subjected to the same one-step RT-PCR as in the case of Fig. 1.
Fig. 3 is a graph which shows the result where lymph nodes (#1 to #2) in which metastasis of cancer was positive by a pathological diagnosis and lymph node (#3) in which metastasis of cancer was negative by a pathological diagnosis were subjected to the one-step RT-PCR of the present invention.

### Summary of the invention

In surgical operations, it is necessary to excise organs and tissues to which cancer metastasizes such as a lymph node in addition to the primary cancer. However, as mentioned herein above already, it is necessary to completely remove the contaminating DNA, etc. and to highly purify the aimed mRNA for the purpose of a precise detection of the expression (mRNA) of a specific gene related to cancer and, therefore, long time is needed for judging the presence or absence of mRNA. On the other hand, there is a limit for the time allowed for a surgical operation. Accordingly, it has not been possible to judge during the operation whether mRNA specific to cancer is present in tissues and organs collected in an operation. Therefore, in order to prevent the recurrence of metastasis of cancer after the operation, it has been necessary to widely excise the tissues and organs to which metastasis is possible such as the lymph nodes without judging whether cancer cells actually had metastasized.

With regard to the above, the burden for a patient is greatly reduced if it is possible to judge within a short time whether an mRNA of a gene related to a specific cancer is present in a sample of tissues and organs collected in a surgical operation. In this case only the tissues and organs in which the mRNA of a cancer-related gene is detected may be excised.

The present invention provides a means where the fact whether the mRNA of CK19 is present in a sample of tissues and organs collected in a surgical operation and whether said tissues and organs are to be excised may be determined within such a short time that the facts may be evaluated during the operation.

The present invention resolves the aforementioned problem by the selection of primers and probes. Thus, the present invention discloses a combination of PCR primers and detection probes by which amplification of the pseudogene is prevented by the selectivity of the PCR primers and, further, the gene is not detected by the selection of the location of the detection probes. Furthermore, when the primers and probes of the present invention are used, there is no necessity of treating a sample for which a PCR is conducted with DNase. Moreover, as will be shown in the Examples, when a nucleic acid synthesizing element having reverse transcription ability is used, the time required for amplification and detection of the CK19 mRNA may be shortened to an extent of around 40 minutes by conducting the reverse transcription step and the PCR step in the same reactor and also by conducting a real-time PCR.

Accordingly, the present invention provides a method for the determination of CK19 mRNA as defined in claim 1, comprising
(a) the amplification of a part of the mRNA using primers, and
(b) the detection of the amplificates formed using two probes,
   wherein said primers are
   - a first primer having a sequence enabling its hybridization to a region located on a first exon of the CK19 gene, and
   - a second primer having a sequence enabling its hybridization to a region located on a second exon of the CK19 gene located downstream to the first exon, and
   wherein said probes are
   - a first probe having a sequence enabling its hybridization to a region located at the 3'-terminus of an exon of the CK19 gene downstream to the region to which said first primer binds and upstream to the region to which said second primer binds,
   - a second probe having a sequence enabling its hybridization to a region located at the 5'-terminus of an adjacent exon of the CK19 gene downstream to the region to which said first probe binds and upstream to the region to which said second primer binds.

In the above-mentioned method, the above-mentioned probes in certain embodiments may be labeled with a donor and an acceptor. For example, the above donor may be FITC and the above acceptor may be selected from the group consisting of rhodamine dyes and cyanine dyes. The aforementioned first probe may in certain embodiments be labeled with a donor at its 3'-terminus and the aforementioned second probe may be labeled with an acceptor at its 5'-terminus. As a specific example, the aforementioned donor is FITC and the aforementioned acceptor is LC-Red 640 which is a rhodamine dye.

Rhodamine dyes are derived from the fusion of phthalic anhydride with m-dialkylaminophenol. Such dyes are disclosed, for example, in EP 567,622 and it is mentioned that pyrano[3,2-g:5,6-g']diguanoline-13-y1,6-(2-carboxy-3,4,5,6-tetrachlorophenyl)-1,11-diethyl-1,2,3,4,8,9,10,11-octa-hydro-2,2,4,8,10,10-hexamethyl perchlorate derivatives may be used as rhodamine dyes.

Cyanine dyes are synthetic dyes having a formula R₂N[CH=CH]ₙCH=N⁺R₂R₂<->R₂N⁺=CH[CH=CH]ₙNR₂ (n is a small numeral). In this formula, nitrogen and conjugated chain moieties usually constitute the part of heterocyclic system such as imidazole, pyridine, pyrrole, quinoline and thiazole. Such dyes are disclosed, for example, in the specifications of US patents 5,268,486, 5,569,587, 5,556,959 and 5,808,044. In certain aspects 1-[3-(4-monomethoxytrityloxy)propyl]-1'-[3-[(2-cyanoethyl)- (N,N-diisopropyl)phosphoramidityl]propyl]-3,3,3',3'-tetra- methyl-4,5-benzindocarbocyanine which is known as Cy3.5 phosphoramidite may be used as a cyanine dye.

The present invention also provides a method for the simultaneous determination of CK19 mRNA and CK20 mRNA as defined in claim 4, comprising
(a) the amplification of a part of the mRNAs using a set of primers for CK19 and a set of primers for CK20, and
(b) the detection of the amplificates formed using a set of probes for CK19 and a
   set of probes for CK20,
   wherein said primers for the CK19 mRNA are
   - a first primer having a sequence enabling its hybridization to a region located on a first exon of the CK19 gene, and
   - a second primer having a sequence enabling its hybridization to a region located on a second exon of the CK19 gene located downstream to the first exon, and
   wherein said probes for the CK19 mRNA are
   - a first probe having a sequence enabling its hybridization to a region located at the 3'-terminus of an exon of the CK19 gene downstream to the region to which said first primer binds and upstream to the region to which said second primer binds, and
   - a second probe having a sequence enabling its hybridization to a region located at the 5'-terminus of an adjacent exon of the CK19 gene downstream to the region to which said first probe binds and upstream to the region to which said second primer binds, and
   wherein said primers for the CK20 mRNA are
   - a third primer having a sequence enabling its hybridization to a region located on the CK20 gene, and
   - a fourth primer having a sequence enabling its hybridization to a region located on the CK20 gene downstream of the third primer, and
   wherein said probes for the CK20 mRNA are
   - a third probe having a sequence enabling its hybridization to a region located on the CK20 gene downstream to the region to which said third primer binds and upstream to the region to which said fourth primer binds, and
   - a fourth probe having a sequence enabling its hybridization to a region located on the CK20 gene upstream to the region to which said fourth primer binds and downstream to the region to which said third probe binds.

In certain embodiments of the above-mentioned method, probes for the detection of the aforementioned CK19 mRNA are labeled with a donor and an acceptor, probes for the detection of the aforementioned CK20 mRNA are labeled with a donor and an acceptor, and, here, the CK19 donor/acceptor pair is different from the aforementioned CK20 donor/acceptor pair. In other embodiments the pair of probes for the detection of the aforementioned CK19 mRNA and the pair of probes for the detection of the aforementioned CK20 mRNA are labeled with the same donor while the acceptor for the detection of the aforementioned CK19 mRNA is different from the acceptor for the detection of the aforementioned CK20 mRNA.

In particular embodiments the aforementioned first probe and third probe are labeled with donors at their 3'-terminus while the aforementioned second probe and fourth probe are labeled with acceptors at their 5'-terminus. For example, the aforementioned donor may be FITC and the aforementioned acceptor may be selected from the group consisting of rhodamine dyes and cyanine dyes. In a specific example, the aforementioned donor is FITC and the aforementioned acceptors are LC-Red 640 and LC-Red 610 which are rhodamine dyes.

In preferred embodiments, the amplification of a part of the aforementioned CK19 mRNA and CK20 mRNA and the detection of the aforementioned amplified products are carried out in the same reaction vessel.

The present invention further provides a composition of matter useful for the detection of CK19 mRN as defined in claim 8, comprising
- a first primer having a sequence enabling its hybridization to a region located on a first exon of the CK19 gene,
- a second primer having a sequence enabling its hybridization to a region located on a second exon of the CK19 gene located downstream to said first exon,
- a first probe having a sequence enabling its hybridization to a region located at the 3'-terminus of an exon of the CK19 gene downstream to the region to which said first primer binds and upstream to the region to which said second primer binds, and
- a second probe having a sequence enabling its hybridization to a region located at the 5'-terminus of an adjacent exon of the CK19 gene downstream to the region to which said first probe binds and upstream to the region to which said second primer binds.

The present invention also provides a primer comprising 10 to 25 base pairs from SEQ ID No. 1 hybridizing to a region located on exon 4 of the CK19 gene, which is the first exon. In certain embodiments the first primer is characterized in being selected from a part of SEQ ID No. 1 containing at least two mismatches to the pseudogene. Preferably, the first primer has exactly the same sequence as SEQ ID No. 2.

The present invention also provides a primer comprising 10 - 25 base pairs from Seq. ID No. 3 hybridizing to a region located on a second exon of the CK19 gene downstream to exon 4. In certain embodiments the above-mentioned primer contains at least two mismatches to the aforementioned CK19 pseudogene. In a particular embodiment the second exon of the aforementioned CK19 gene is exon 6. Preferably, the aforementioned second primer has exactly the same sequence as SEQ ID No. 4.

The present invention also provides a combination of primers comprising at least one primer according to anyone of the above primers useful for the amplification of a part of the CK19 mRNA. In preferred embodiments the aforementioned at least one primer sequence contains at least one mismatch to the CK19 pseudogene at its 3'-terminus.

The present invention also provides a first probe comprising 10 to 25 base pairs from SEQ ID No. 5, which is the complete sequence of CK19 mRNA, hybridizing to a region located at the 3'-terminus of a first exon of the CK19 gene. In certain embodiments the aforementioned first probe hybridizes to an amplificate prepared by the above-mentioned primer combination. In certain aspects the first exon of the aforementioned CK19 gene is exon 4. For example, the exact sequence of the aforementioned first probe is SEQ ID No. 6.

The present invention further provides a second probe comprising 10 to 25 base pairs from SEQ ID No. 5, which is the complete sequence of CK19 mRNA, hybridizing to a region located at the 5'-terminus of a second exon of the CK19 gene. In certain embodiments the aforementioned second probe hybridizes to an exon located downstream the above-mentioned first probe and being adjacent on an amplificate prepared by the above-mentioned primer combination. In certain aspects the exon located downstream of the first exon of the CK19 gene is exon 5. For example, the exact sequence of the aforementioned second probe is SEQ ID No. 7.

The present invention also relates to combinations of the above-mentioned probes useful for identification of a CK19 mRNA amplificate. The probes may be labeled with a donor and an acceptor. In certain aspects of the combination of probes the aforementioned donor is FITC and the aforementioned acceptor is selected from the group consisting of rhodamine dyes and cyanine dyes. In another embodiment of the combination of probes the aforementioned first probe is labeled with a donor at its 3'-terminus and the second probe bonding in an adjacent manner downstream to the aforementioned first probe is labeled with an acceptor at its 5'-terminus. In that case, for example, the aforementioned donor is FITC and the aforementioned acceptor is a rhodamine dye, e.g., LC-Red 640.

The present invention also provides a kit for the amplification and detection of CK19 mRNA, wherein said kit at least comprises a pair of primers with
- a first primer having a sequence enabling its hybridization to a region located on a first exon of the CK19 gene, and
- a second primer having a sequence enabling its hybridization to a region located on a second exon of the CK19 gene located downstream to said first exon, and
a pair of probes with
- a first probe hybridizing to a region located at the 3'-terminus of an exon of the CK19 gene downstream to the region to which said first primer binds and upstream to the region to which said second primer binds, and
- a second probe hybridizing to a region located at the 5'-terminus of an adjacent exon of the CK19 gene downstream to the region to which said first probe binds and upstream to the region to which said second primer binds.

In certain aspects of the kit the aforementioned primer pair is present as a mixture or the aforementioned probe pair is present as a mixture or both of the aforementioned primer pair and the aforementioned probe pair are present as a mixture. The aforementioned kit may further contain a buffer solution.

The present invention also provides a kit for the combined amplification and detection of CK19 mRNA and CK20 mRNA. Such a kit at least comprises two pairs of primers and two pairs of probes, wherein the aforementioned probes are labeled differently for CK19 and CK20. In certain embodiments of the kit, said primers and probes for the amplification and the detection of CK19 mRNA comprise
- a first primer having a sequence enabling its hybridization to a region located on a first exon of the CK19 gene,
- a second primer having a sequence enabling its hybridization to a region located on a second exon of the CK19 gene located downstream to said first exon,
- a first probe hybridizing to a region located at the 3'-terminus of an exon of the CK19 gene downstream to the region to which said first primer binds and upstream to the region to which said second primer binds, and
- a second probe hybridizing to a region located at the 5'-terminus of an adjacent exon of the CK19 gene downstream to the region to which said first probe binds and upstream to the region to which said second primer binds.

In certain aspects of the above-mentioned kit, the aforementioned primer pair is present as a mixture or the aforementioned probe pair is present as a mixture or both of the aforementioned primer pair and the aforementioned probe pair are present as a mixture. The aforementioned kit may in certain embodiments further contain a buffer solution.

The present invention provides novel PCR primers and probes for the specific amplification and detection of CK19 mRNA by real-time RT-PCR and hybridization probe techniques, particularly useful for clinical testing. Furthermore, the reaction conditions therefore are provided.

In order to achieve an RT-PCR which is highly specific to CK19 mRNA even when human DNA is contaminating a sample, the region and positions of PCR primers and detection probes were determined on the basis of the following designs:
1) A design is carried out so that, in RT-PCR, each 3'-terminus of the two PCR primers comprises mismatch sites of the pseudogene of CK19 and CK19 mRNA whereby amplification of the CK19 pseudogene is not possible even when amplification of CK 19 mRNA is possible.
2) With regard to the region to be subjected to gene amplification, the site which satisfies the above-mentioned condition and which contains at least one boundary of exon-intron of the CK19 gene is selected.
3) When two probes for detection hybridize to a target sequence on the basis of a hybridization probe technique, the design is done in such a manner that the aforementioned exon-intron boundary is located between the hybridizing positions of the two probes for detection. The design may in other cases also be done in such a manner that one of the two probes for detection hybridizes onto an exon-intron boundary whereby an amplificate derived from the CK19 gene is not identified.

### 1) Design of PCR primer

The PCR primers provided by the present invention complementarily hybridize to CK19 mRNA. If the PCR primers could also hybridize to the CK19 pseudogene, the setting is deferred to a base region where the 3'-terminus of the PCR primers is not complementary to at least one base or preferably two bases.

Since the present invention is applied to clinical diagnosis, PCR primers are designed so as to make a real-time PCR possible. To be more specific, the size of the PCR primers should be 10 to 25 bases or, preferably, 17 to 18 bases. With regard to GC%, it is preferred to be 45% to 55%. The melting temperature, Tm, should be 45°C to 55°C or, preferably, around 52°C.

In order to shorten the PCR amplifying time, the size of the amplificate amplified by the PCR is preferred to be 150 to 250 base pairs. In addition, one or more exon-intron boundary/boundaries of the gene must be present in the region which is amplified by the PCR.

In a region which is amplified by the PCR, a site to which a probe for detection hybridizes is installed.

### 2) Design of probes for detection

With regard to the two kinds of probes for detection provided by the present invention, each of them complimentarily hybridizes to CK19 mRNA amplified by the PCR.

Two kinds of probes for detection are DNA oligonucleotides labeled with different fluorescent dyes and one is called a donor probe while another is called an acceptor probe. In the donor probe, its 5'-terminus is labeled with a fluorescent dye while, in the acceptor probe, its 3'-terminus is subjected to a fluorescent labeling.

Those two probes for detection are designed so as to hybridize to very near regions which are apart to an extent of only 1 to 5 base(s). As a result thereof, the so-called fluorescence resonance energy transfer (FRET) occurs between fluorescent dyes labeled on two probes. To be more specific, when light which excites the fluorescent dye of donor probe is irradiated, the excited energy transfers to the fluorescent dye on the adjacent acceptor probe whereupon fluorescence is generated. Such a phenomenon occurs only when the two probes hybridize to the exactly adjacent places.

The size of the probe for detection is between 10 to 25 bases or, preferably, 18 to 22 bases. GC% is preferred to be 45% to 55%. The melting temperature should be 50°C to 65°C and is set to be 5°C higher than the melting temperature of the primers and, furthermore, the melting temperature of the acceptor probe is set to be 2 to 3°C higher than the melting temperature of the donor probe.

### 3) RT-PCR method and reaction protocol

The PCR using the PCR primers and detection probes provided by the present invention may be carried out in a thermal cycler where all real-time PCRs are possible. At that time, the temperature control property of each machine and the corresponding fluorescence filter are different and, therefore, upon necessity, optimization of the PCR conditions and the selection of fluorescent dyes are required. Here in the Examples, the PCR conditions will be illustrated when a Light Cycler® is used (distributor: Roche Diagnostic K. K.).

In carrying out the RT-PCR, mRNA which is the target, is firstly subjected to a reverse transcription to synthesize cDNA and then the PCR is conducted. The PCR using the PCR primers and detection probes provided by the present invention may be used when the reverse transcription step and the PCR step are divided (two-step RT-PCR). In other applications it is also possible to use the primers in case where the reverse transcription step and the PCR step are conducted in one reaction vessel (one-step RT-PCR). Here in the Examples, the result of a one-step RT-PCR which is quicker is shown.

### 4) Method for detection of PCR amplificate

In order to use the detection probes provided by the present invention, it is necessary to use a thermal cycler which is equipped with a light source generating the wavelength which is able to excite the fluorescent dye of the donor probes and which is also equipped with a detector able to measure the fluorescence of the fluorescent dye of the acceptor probes.

Here in Examples, the PCR conditions are shown when a Light Cycler^{®} (distributor: Roche Diagnostic K. K.) is used.

### 5) Reagent and reagent kit

The PCR primers and the detection probes of the present invention may also be provided in a kit together with reagents which are necessary for amplification and detection of mRNA of the CK19 gene as the target.

### Examples

Examples of the present invention will be shown as follows although the present invention is not limited to those Examples only.

### Example 1: Design of PCR primers

As mentioned already, the pseudogene of a process type has been report for CK19. It is necessary to prepare PCR primers which are not affected even when such a pseudogene is contaminating the sample.

For such a purpose, three kinds of forward primers (F1 to F3) and two kinds of reverse primers (R1 to R2) were prepared. F1 to F3 and R1 were designed in such a manner that their 3'-terminus exhibit a mismatch site of the CK19 pseudogene and the CK19 mRNA. R2 was designed in such a manner that a boundary of intron-exon is present in its base sequence. F1 and R1 contain two mismatches to the CK19 pseudogene and F2, F3 and R2 have one mismatch to the CK19 pseudogene.
F1: TGAGTGACATGCGAAGC (799 to 815 of bases of SEQ ID No. 5) (SEQ ID No. 2)
F2: CGCCAAGATCCTGAGTG (788 to 804 of bases of SEQ ID No. 5) (SEQ ID No. 8)
F3: GACATGCGAAGCCAATAT (804 to 821 of bases of SEQ ID No. 5) (SEQ ID No. 9)
R1: TGTGTCTTCCAAGGCA (1007 to 1022 of bases of SEQ ID No. 5) (SEQ ID No. 4)
R2: CCAAGGCAGCTTTCAT (999 to 1014 of bases of SEQ ID No. 5) (SEQ ID No. 10)

PCR was carried out under the following conditions using primer combinations of F1/R1, F1/R2, F2/R2 and F3/R2.

With regard to an enzyme used for the reaction (Tth DNA polymerase), a commercially available kit (Light Cycler^{®} RNA Master Hybridization Probes; distributor: Roche Diagnostic K. K.) was used.

| | |
|---|---|
| 50 mM manganese acetate | 3.25 mM |
| PCR primers | 0.25 µM each |
| Tth DNA polymerase | 7.5 µl/reaction |
| CK19 mRNA or CK19 pseudogene | 10⁵ copies/PCR |

A reaction solution (20 µl) containing the above components was placed in a glass capillary and subjected to a one-step RT-PCR using a Light Cycler^{®} under the condition as shown in Table 1 and 5 µl of the reaction product were analyzed by 3% agarose gel electrophoresis.

**Table 1:**

| Program 1: RT | | | |
|---|---|---|---|
| Cycle Program Data | Value | | |
| Cycles | 1 | | |
| Analysis Mode | None | | |
| Temperature Targets | Segment 1 | | |
| Target Temperature (°C) | 61 | | |
| Incubation time (h:min:s) | 00:05:00 | | |
| Temperature Transition Rate (°C/s) | 20.0 | | |
| Secondary Target Temperature (°C) | 0 | | |
| Step Size (°C) | 0.0 | | |
| Step Delay (Cycles) | 0 | | |
| Acquisition Mode | None | | |
| | | | |

| Program 2: Denaturation | | | |
|---|---|---|---|
| Cycle Program Data | Value | | |
| Cycles | 1 | | |
| Analysis Mode | None | | |
| Temperature Targets | Segment 1 | | |
| Target Temperature (°C) | 95 | | |
| Incubation time (h:min:s) | 00:00:30 | | |
| Temperature Transition Rate (°C/s) | 20.0 | | |
| Secondary Target Temperature (°C) | 0 | | |
| Step Size (°C) | 0.0 | | |
| Step Delay (Cycles) | 0 | | |
| Acquisition Mode | None | | |
| | | | |

| Program 3: Amplification | | | |
|---|---|---|---|
| Cycle Program Data | Value | | |
| Cycles | 40 | | |
| Analysis Mode | None | | |
| Temperature Targets | Segment 1 | Segment 2 | Segment 3 |
| Target Temperature (°C) | 95 | 45 | 72 |
| Incubation time (h:min:s) | 00:00:01 | 00:00:15 | 00:00:05 |
| Temperature Transition Rate (°C/s) | 20.0 | 20.0 | 2.0 |
| Secondary Target Temperature (°C) | 0 | 0 | 0 |
| Step Size (°C) | 0.0 | 0.0 | 0.0 |
| Step Delay (Cycles) | 0 | 0 | 0 |
| Acquisition Mode | None | Single | None |
| | | | |

| Program 4: Cooling | | | |
|---|---|---|---|
| Cycle Program Data | Value | | |
| Cycles | 1 | | |
| Analysis Mode | None | | |
| Temperature Targets | Segment 1 | | |
| Target Temperature (°C) | 40 | | |
| Incubation time (h:min:s) | 00:00:30 | | |
| Temperature Transition Rate (°C/s) | 20.0 | | |
| Secondary Target Temperature (°C) | 0 | | |
| Step Size (°C) | 0.0 | | |
| Step Delay (Cycles) | 0 | | |
| Acquisition Mode | None | | |

In F1/R1 (in both F1 and R1, two mismatches to the CK19 pseudogene are present), although some bands supposed to be amplificates from the CK19 gene were noted, no amplificate from CK19 pseudogene was noted at all.

In F1/R2, F2/R2 and F3/R2 (in all of R2, F2 and F3, one mismatch to the CK19 pseudogene is present), although no band supposed to be amplificates from the CK19 gene were noted, amplificate from CK19 pseudogene was noted a little.

It was judged to be difficult not to detect the amplificate from the CK19 pseudogene by improvement in the detection probes whereby F1/R1 where no amplificate from CK19 pseudogene was noted was chosen to be used further.

### Example 2: Design of detection probes

Two sets of detection probes were prepared and compared. With regard to donor probes (P1 and P1b), their 3'-terminus was labeled with FITC while, with regard to acceptor probes, their 5'-terminus was labeled with LC-Red 460.

### Set 1

P1: GTCATGGCCGAGCAGAACC (825 to 843 of bases of SEQ ID No. 5) (SEQ ID No. 11)
P2: AAGGATGCTGAAGCCTGGT (846 to 864 of bases of SEQ ID No. 5) (SEQ ID No. 12)

### Set 2

P1b: AAGCCTGGTTCACCAGCCG (856 to 874 of bases of SEQ ID No. 5) (SEQ ID No. 6)
P2c: CTGAAGAATTGAACCGGGAGG (877 to 897 of bases of SEQ ID No. 5) (SEQ ID No. 7)

Set 2 was designed in such a manner that a boundary of exon-intron is located between the hybridization positions of the two probes while set 1 was not designed as such.

For evaluation of probe sets, 20 µl of a reaction solution containing the following components was placed in a glass capillary and subjected to a one-step RT-PCR using a Light Cycler® under the condition as shown in Table 1.

| | |
|---|---|
| 50 mM manganese acetate | 3.25 mM |
| PCR primers F1 and R1 | 0.25 µM each |
| Donor probe (P1 or P1b) | 25 nM |
| Acceptor probe (P1 or P2c) | 100 nM |
| Tth DNA polymerase | 7.5 µl/reaction |
| CK19 mRNA or CK19 pseudogene | 10⁵ copies/PCR |

As a result, fluorescent signal due to amplification of the CK19 gene was noted in set 1 while, in set 2, no fluorescent signal was noted at all.

From the above investigations, there was established a combination of primers (F1 and R1) and probes (P1b and P2c) where amplification of the CK19 pseudogene was excluded by selection of the PCR primers and amplification signal of the CK19 gene was excluded by selection of the detection probes.

### Example 3: Real-time RT-PCR of CK19 mRNA

An example of a real-time RT-PCR of CK19 mRNA which is a positive control and human DNA and CK19 pseudogene is shown.

CK19 mRNA was diluted 10-fold in a stepwise manner and 10⁵ to 10² copies were subjected to a one-step RT-PCR amplification using a Light Cycler^{®}. Similarly, 500 ng of human DNA or 10⁵ copies of CK19 pseudogene were amplified and detected by RT-PCR (time required was about 40 minutes). The compositions of the reaction solution (20 µl/PCR) were as follows below.

| | |
|---|---|
| 50 mM manganese acetate | 3.25 mM |
| PCR primers F1 and R1 | 0.25 µM each |
| Donor probe P1b | 25 nM |
| Acceptor probe P2c | 100 nM |
| Tth DNA polymerase | 7.5 µl/reaction |

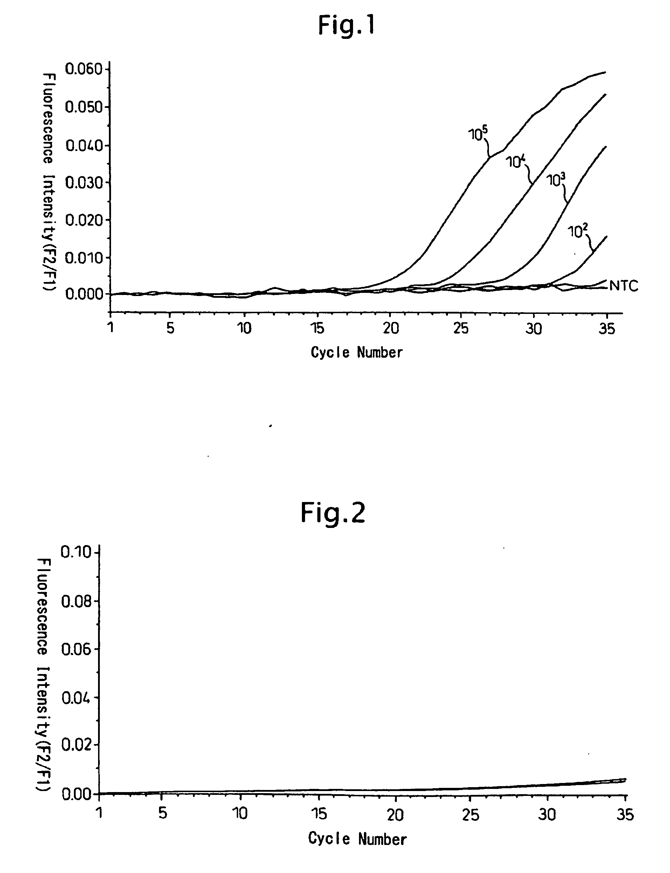

The results are shown in Fig. 1 and Fig. 2. In those drawings, an ordinate shows intensity of fluorescence while an abscissa shows PCR cycle numbers.

When CK19 mRNA was amplified by RT-PCR, fluorescent signals were generated in PCR cycle numbers depending upon the initial amount (Fig. 1) while, when human DNA or CK19 pseudogene was subjected to PCR similarly, no such fluorescent signal was noted (Fig. 2).

### Example 4: Detection of CK19 mRNA in human lymph node

An example of real-time RT-PCR of CK19 mRNA using a clinical sample is shown. Among lymph nodes excised from a patient suffering from stomach cancer of cT1NO, the lymph nodes where metastasis of cancer was noted by a pathological diagnosis and the lymph nodes where no metastasis of cancer was noted were selected and homogenized in a buffer solution (600 µl) containing guanidine thiocyanate. The homogenization was carried out using a MagNA Lyser^{®} (distributor: Roche Diagnostic K. K.). Distilled water (500 µl) was added to 450 µl of the homogenate and centrifuged at 14500 × g and the supernatant liquid (900 µl) was transferred to a new tube. To this 2 µl of an oligonucleotide (biotin-5'-gcttcacatccctccgctgattctcttga) labeled with 5 µM of biotin was added and the mixture was allowed to stand at 37°C for 10 minutes whereupon the oligonucleotide and CK19 mRNA hybridized.

After that, magnetic particles coated with Avidin were added thereto and the mixture was allowed to stand at 37°C for 10 minutes more whereupon an mRNA-oligonucleotide complex was trapped on the magnetic particles. The magnetic particles were washed with a buffer solution containing a surfactant twice to remove excessive components, then 50 µl of a TE buffer solution was added so that the magnetic particles were well suspended therein and CK19 mRNA on the magnetic particles was extracted with heat (time required was about 45 minutes).

The extracted sample (2 µl) was subjected to a one-step RT-PCR. The specific conditions therefore were the same as in Example 3 (time required was about 40 minutes).

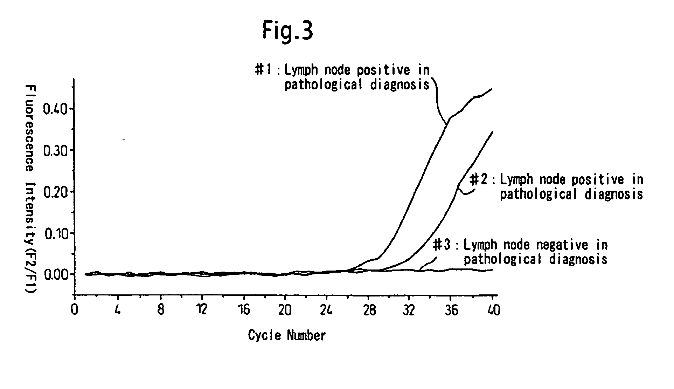

The result is shown in Fig. 3. The lymph nodes (#1 to #2) where metastasis of cancer was positive in the pathological diagnosis were positive in the RT-PCR while the lymph node (#3) where metastasis of cancer was negative in the pathological diagnosis was negative in the RT-PCR.

### Example 5: An example of multiplex real-time RT-PCR

In clinical tests, plural test items are sometimes tested by one measurement. This is important for saving costs and labor. Especially when diagnosis for presence or absence of cancer cells and for metastasis is conducted, judgment by the result of one single marker only has a certain possibility of risk of a wrong diagnosis. Accordingly, if other markers in addition to CK19 mRNA may be subjected to a real-time RT-PCR at the same time, the usefulness is enhanced in applying the test to clinical diagnosis.

Now, mRNA of CK20 which is the same kind of cytokeratin as CK19 was subjected to amplification together with CK19 mRNA. Furthermore, a protocol for a separate detection was established. Although the conditions for RT-PCR were the same as that in the aforementioned Example 3, PCR primers (CK20F and CK20R) and detection primers (CK20P1 and CK20P2) for CK20 were added to the reaction solution. In the detection probes, 3'-terminus was labeled with FITC for the donor probes while, for acceptor probes, 5'-terminus was labeled with LC-Red 610 for discriminating from the detection wavelength of CK19.
CK20F: ATCAAGCAGTGGTACGAAAC (SEQ ID No. 13)
CK20R: AGGACACACCGAGCATTT (SEQ ID No. 14)
CK20P1: ATTACAGACAAATTGAAGAGCTGCC (SEQ ID No. 15)
CK20P2: AGTCAGATTAAGGATGCTCAACTGC (SEQ ID No. 16)

| | |
|---|---|
| 50 mM manganese acetate | 3.25 mM |
| CK19 PCR primers F1 and R1 | 0.25 µM each |
| CK19 donor probe P1b | 25 nM |
| CK19 acceptor probe P2c | 100 nM |
| CK20 PCR primers CK20F and CK20R | 0.25 µM each |
| CK20 donor probe CK20P1 | 25nM |
| CK20 acceptor probe CK20P2 | 100 nM |
| Tth DNA polymerase | 7.5 µl/reaction |
| Positive control, mRNA of CK19 gene | |
| Positive control, mRNA of CK20 gene | |

Both, mRNA for CK19 and CK20 were amplified in the same tube and their amplifications were separately monitored due to their different detection wavelength (640 nm for CK19 and 610 nm for CK20).

## Claims

1. A method for the determination of CK19 mRNA comprising
(a) the amplification of a part of the mRNA using primers, and
(b) the detection of the amplificates formed using two probes;
wherein said primers are
- a first primer comprising SEQ ID NO:2, and
- a second primer comprising SEQ ID NO:4;
wherein said probes are
- a first probe having a sequence enabling its hybridization to a region located at the 3'-terminus of an exon of the CK19 gene downstream to the region to which said first primer binds and upstream to the region to which said second primer binds, and
- a second probe having a sequence enabling its hybridization to a region located at the 5'-terminus of an adjacent exon of the CK19 gene downstream to the region to which said first probe binds and upstream to the region to which said second primer binds;
one of said probes being labeled with a donor and the other of said probes being labeled with an acceptor.

2. The method according to claim 1, wherein the donor is FITC (fluorescein isothiocyanate) and the acceptor is selected from a group consisting of rhodamine dyes and cyanine dyes.

3. The method according to claim 1 or 2, wherein the first probe is labeled with a donor at its 3'-terminus and wherein the second probe is labeled with an acceptor at its 5'-terminus.

4. A method for the simultaneous determination of CK19 mRNA and CK20 mRNA comprising
(a) the amplification of a part of the mRNAs using a set of primers for CK19 and a set of primers for CK20, and
(b) the detection of the amplificates formed using a set of probes for CK19 and a set of probes for CK20;
wherein said primers for the CK19 mRNA are
- a first primer comprising SEQ ID NO:2, and
- a second primer comprising SEQ ID NO:4; ;
wherein said probes for the CK19 mRNA are
- a first probe having a sequence enabling its hybridization to a region located at the 3'-terminus of an exon of the CK19 gene downstream to the region to which said first primer binds and upstream to the region to which said second primer binds, and
- a second probe having a sequence enabling its hybridization to a region located at the 5'-terminus of an adjacent exon of the CK19 gene downstream to the region to which said first probe binds and upstream to the region to which said second primer binds;
wherein said primers for the CK20 mRNA are
- a third primer having a sequence enabling its hybridization to a region located on the CK20 gene, and
- a fourth primer having a sequence enabling its hybridization to a region located on the CK20 gene downstream of the third primer; and
wherein said probes for the CK20 mRNA are
- a third probe having a sequence enabling its hybridization to a region located on the CK20 gene downstream to the region to which said third primer binds and upstream to the region to which said fourth primer binds, and
- a fourth probe having a sequence enabling its hybridization to a region located on the CK20 gene upstream to the region to which said fourth primer binds and downstream to the region to which said third probe binds;
wherein the probes for the detection of CK19 are labeled with a donor and an acceptor and the probes for the detection of CK20 are labeled with a donor and an acceptor and wherein the CK19 donor/acceptor pair is differing from the CK20 donor/acceptor pair.

5. The method according to claim 4, wherein the set of probes for the detection of CK19 and the set of probes for the detection of CK20 contain the same donor, while the acceptor for the detection of CK19 is differing from the acceptor for the detection of CK20.

6. The method according to claims 4 or 5, wherein the first and the third probe are labeled with a donor at their 3'-terminus and wherein the second and the fourth probe are labeled with an acceptor at their 5'-terminus.

7. The method according to any of claims 4 to 6, wherein the donor is FITC and the acceptor is selected from a group consisting of rhodamine dyes and cyanine dyes.

8. A kit for the amplification and detection of CK19 mRNA, wherein said kit at least comprises
a pair of primers with
- a first primer comprising SEQ ID NO:2, and
- a second primer comprising SEQ ID NO:4;
as well as a pair of probes with
- a first probe hybridizing to a region located at the 3'-terminus of an exon of the CK19 gene downstream to the region to which said first primer binds and upstream to the region to which said second primer binds, and
- a second probe hybridizing to a region located at the 5'-terminus of an adjacent exon of the CK19 gene downstream to the region to which said first probe binds and upstream to the region to which said second primer binds:
one of said probes being labeled with a donor and the other of said probes being labeled with an acceptor.

9. The kit according to claim 8 for the combined amplification and detection of CK19 mRNA and CK20 mRNA further comprising at least a second pair of primers and a second pair of probes, the probes for the detection of CK19 being labeled with a donor and an acceptor and the probes for the detection of CK20 being labeled with a donor and an acceptor, wherein the CK19 donor/acceptor pair is differing from the CK20 donor/acceptor pair.

10. The kit according to claim 9, wherein said primers for the CK20 mRNA are
- a third primer having a sequence enabling its hybridization to a region located on the CK20 gene, and
- a fourth primer having a sequence enabling its hybridization to a region located on the CK20 gene downstream of the third primer; and
wherein said probes for the CK20 mRNA are
- a third probe having a sequence enabling its hybridization to a region located on the CK20 gene downstream to the region to which said third primer binds and upstream to the region to which said fourth primer binds, and
- a fourth probe having a sequence enabling its hybridization to a region located on the CK20 gene upstream to the region to which said fourth primer binds and downstream to the region to which said third probe binds.

11. The kit according to claim 8 to 10, wherein the pairs of primers are contained in a mixture or wherein the pairs of probes are contained in a mixture or wherein the pairs of primers and the pairs of probes are contained in a mixture.

12. The kit according to any one of claims 8 to 11 further containing a buffer solution.

## Patentansprüche

1. Verfahren zur Bestimmung von CK19-mRNA, das Folgendes umfasst:
(a) Amplifizieren eines Teils der mRNA unter Verwendung von Primern, und
(b) Nachweisen der gebildeten Amplifikate unter Einsatz von zwei Sonden;
wobei es sich bei den Primern um
- einen ersten Primer, umfassend SEQ ID NO: 2, und
- einen zweiten Primer, umfassend SEQ ID NO: 4, handelt;
wobei es sich bei den Sonden um
- eine erste Sonde mit einer Sequenz, die es der Sonde gestattet, an eine Region zu hybridisieren, die sich am 3'-terminalen Ende eines Exons des CK19-Gens stromabwärts von der Region, an die der erste Primer bindet, und stromaufwärts von der Region, an die der zweite Primer bindet, befindet, und
- eine zweite Sonde mit einer Sequenz, die es der Sonde gestattet, an eine Region zu hybridisieren, die sich am 5'-terminalen Ende eines benachbarten Exons des CK19-Gens stromabwärts von der Region, an die die erste Sonde bindet, und stromaufwärts von der Region, an die der zweite Primer bindet, befindet,
handelt,
wobei eine der Sonden mit einem Donor markiert ist und die andere der Sonden mit einem Akzeptor markiert ist.

2. Verfahren nach Anspruch 1, wobei es sich bei dem Donor um FITC (Fluoresceinisothiocyanat) handelt und der Akzeptor aus einer Gruppe bestehend aus Rhodaminfarbstoffen und Cyaninfarbstoffen ausgewählt ist.

3. Verfahren nach Anspruch 1 oder 2, wobei die erste Sonde mit einem Donor an ihrem 3'-terminalen Ende markiert ist und die zweite Sonde mit einem Akzeptor an ihrem 5'-terminalen Ende markiert ist.

4. Verfahren für die gleichzeitige Bestimmung von CK19-mRNA und CK20-mRNA, umfassend:
(a) das Amplifizieren eines Teils der mRNAs unter Verwendung eines Primer-Satzes für CK19 und eines Primer-Satzes für CK20 und
(b) Nachweisen der gebildeten Amplifikate unter Verwendung eines Sondensatzes für CK19 und eines Sondensatzes für CK20;
wobei es sich bei den Primern für die CK19-mRNA um
- einen ersten Primer, umfassend SEQ ID NO: 2, und
- einen zweiten Primer, umfassend SEQ ID NO: 4, handelt;
wobei es sich bei den Sonden für die CK19-mRNA um
- eine erste Sonde mit einer Sequenz, die es der Sonde gestattet, an eine Region zu hybridisieren, die sich am 3'-terminalen Ende eines Exons des CK19-Gens stromabwärts von der Region, an die der erste Primer bindet, und stromaufwärts von der Region, an die der zweite Primer bindet, befindet, und
- eine zweite Sonde mit einer Sequenz, die es der Sonde gestattet, an eine Region zu hybridisieren, die sich am 5'-terminalen Ende eines benachbarten Exons des CK19-Gens stromabwärts von der Region, an die die erste Sonde bindet, und stromaufwärts von der Region, an die der zweite Primer bindet, befindet,
handelt,
wobei es sich bei den Primern für die CK20-mRNA um
- einen dritten Primer mit einer Sequenz, die es ihm gestattet, an eine Region, die sich auf dem CK20-Gen befindet, zu hybridisieren, und
- einen vierten Primer mit einer Sequenz, die es ihm gestattet, an eine Region, die sich auf dem CK20-Gen stromabwärts des dritten Primers befindet, zu hybridisieren,
handelt; und
wobei es sich bei den Sonden für die CK20-mRNA um
- eine dritte Sonde mit einer Sequenz, die es ihr gestattet, an eine Region zu hybridisieren, die sich auf dem CK20-Gen stromabwärts von der Region, an die der dritte Primer bindet, und stromaufwärts von der Region, an die der vierte Primer bindet, befindet, und
- eine vierte Sonde mit einer Sequenz, die es ihr gestattet, mit einer Region zu hybridisieren, die sich auf dem CK20-Gen stromaufwärts von der Region, an die der vierte Primer bindet, und stromabwärts von der Region, an die die dritte Sonde bindet, befindet,
handelt, wobei die Sonden für den Nachweis von CK19 mit einem Donor und einem Akzeptor markiert sind und die Sonden für den Nachweis von CK20 mit einem Donor und einem Akzeptor markiert sind und wobei sich das CK19-Donor/Akzeptorpaar von dem CK20-Donor/Akzeptorpaar unterscheidet.

5. Verfahren nach Anspruch 4, wobei der Satz von Sonden für den Nachweis von CK19 und der Satz von Sonden für den Nachweis von CK20 denselben Donor enthalten, während sich der Akzeptor für den Nachweis von CK19 von dem Akzeptor für den Nachweis von CK20 unterscheidet.

6. Verfahren nach den Ansprüchen 4 oder 5, wobei die erste und die dritte Sonde an ihrem 3'-terminalen Ende mit einem Donor markiert sind und wobei die zweite und die vierte Sonde an ihrem 5'-terminalen Ende mit einem Akzeptor markiert sind.

7. Verfahren nach einem der Ansprüche 4 bis 6, wobei es sich bei dem Donor um FITC handelt und der Akzeptor aus der Gruppe bestehend aus Rhodaminfarbstoffen und Cyaninfarbstoffen ausgewählt ist.

8. Kit zum Amplifizieren und Nachweisen von CK19-mRNA, wobei das Kit mindestens
ein Primerpaar, wobei
- ein erster Primer SEQ ID NO: 2 umfasst und
- ein zweiter Primer SEQ ID NO: 4 umfasst;
sowie ein Sondenpaar, wobei
- eine erste Sonde mit einer Region hybridisiert, die sich am 3'-terminalen Ende eines Exons des CK19-Gens stromabwärts von der Region, an die der erste Primer bindet, und stromaufwärts von der Region, an die der zweite Primer bindet, befindet, und
- eine zweite Sonde mit einer Region hybridisiert, die sich am 5'-terminalen Ende eines benachbarten Exons des CK19-Gens stromabwärts von der Region, an die die erste Sonde bindet, und stromaufwärts von der Region, an die der zweite Primer bindet, befindet;
wobei eine der Sonden mit einem Donor markiert ist und die andere der Sonden mit einem Akzeptor markiert ist,
umfasst.

9. Kit nach Anspruch 8 für das kombinierte Amplifizieren und Nachweisen von CK19-mRNA und CK20-mRNA, das weiterhin mindestens ein zweites Primerpaar und ein zweites Sondenpaar umfasst, wobei die Sonden für den Nachweis von CK19 mit einem Donor und einem Akzeptor markiert sind und die Sonden für den Nachweis von CK20 mit einem Donor und einem Akzeptor markiert sind, wobei sich das CK19-Donor/Akzeptorpaar von dem CK20-Donor/Akzeptorpaar unterscheidet.

10. Kit nach Anspruch 9, wobei es sich bei den Primern für die CK20-mRNA um
- einen dritten Primer mit einer Sequenz, die es ihm gestattet, an eine Region zu hybridisieren, die sich auf dem CK20-Gen befindet, und
- einen vierten Primer mit einer Sequenz, die es ihm gestattet, an eine Region zu hybridisieren, die sich auf dem CK20-Gen stromabwärts des dritten Primers befindet, handelt; und
wobei es sich bei den Sonden für die CK20-mRNA um
- eine dritte Sonde mit einer Sequenz, die es ihr gestattet, an eine Region zu hybridisieren, die sich auf dem CK20-Gen stromabwärts von der Region, an die der dritte Primer bindet, und stromaufwärts von der Region, an die der vierte Primer bindet, befindet, und
- eine vierte Sonde mit einer Sequenz, die es ihr gestattet, an eine Region zu hybridisieren, die sich auf dem CK20-Gen stromaufwärts von der Region, an die der vierte Primer bindet, und stromabwärts von der Region, an die die dritte Sonde bindet, befindet,
handelt.

11. Kit nach den Ansprüchen 8 bis 10, wobei das Primerpaar in einer Mischung enthalten ist oder wobei das Sondenpaar in einer Mischung enthalten ist oder wobei das Primerpaar und das Sondenpaar in einer Mischung enthalten sind.

12. Kit nach einem der Ansprüche 8 bis 11, das weiterhin eine Pufferlösung enthält.

## Revendications

1. Procédé pour la détermination de l'ARNm de CK19 comprenant
(a) l'amplification d'une partie de l'ARNm en utilisant des amorces, et
(b) la détection des produits d'amplification formés en utilisant deux sondes ;
dans lequel lesdites amorces sont
- une première amorce comprenant SEQ ID NO: 2, et
- une deuxième amorce comprenant SEQ ID NO: 4 ;
dans lequel lesdites sondes sont
- une première sonde ayant une séquence permettant son hybridation à une région située à l'extrémité 3' d'un exon du gène de CK19 en aval de la région à laquelle ladite première amorce se lie et en amont de la région à laquelle ladite deuxième amorce se lie, et
- une deuxième sonde ayant une séquence permettant son hybridation à une région située à l'extrémité 5' d'un exon adjacent au gène de CK19 en aval de la région à laquelle ladite première sonde se lie et en amont de la région à laquelle ladite deuxième amorce se lie ;
l'une desdites sondes étant marquée avec un donneur et l'autre desdites sondes étant marquée avec un accepteur.

2. Procédé selon la revendication 1, dans lequel le donneur est le FITC (isothiocyanate de fluorescéine) et l'accepteur est choisi dans un groupe constitué par les colorants de type rhodamine et les colorants de type cyanine.

3. Procédé selon la revendication 1 ou 2, dans lequel la première sonde est marquée avec un donneur à son extrémité 3', et dans lequel la deuxième sonde est marquée avec un accepteur à son extrémité 5'.

4. Procédé pour la détermination simultanée de l'ARNm de CK19 et l'ARNm de CK20 comprenant
(a) l'amplification d'une partie des ARNm en utilisant un jeu d'amorces pour CK19 et un jeu d'amorces pour CK20, et
(b) la détection des produits d'amplification formés en utilisant un jeu de sondes pour CK19 et un jeu de sondes pour CK20 ;
dans lequel lesdites amorces pour l'ARNm de CK19 sont
- une première amorce comprenant SEQ ID NO: 2, et
- une deuxième amorce comprenant SEQ ID NO: 4 ;
dans lequel lesdites sondes pour l'ARNm de CK19 sont
- une première sonde ayant une séquence permettant son hybridation à une région située à l'extrémité 3' d'un exon du gène de CK19 en aval de la région à laquelle ladite première amorce se lie et en amont de la région à laquelle ladite deuxième amorce se lie, et
- une deuxième sonde ayant une séquence permettant son hybridation à une région située à l'extrémité 5' d'un exon adjacent au gène de CK19 en aval de la région à laquelle ladite première sonde se lie et en amont de la région à laquelle ladite deuxième amorce se lie ;
dans lequel lesdites amorces pour l'ARNm de CK20 sont
- une troisième amorce ayant une séquence permettant son hybridation à une région située sur le gène de CK20, et
- une quatrième amorce ayant une séquence permettant son hybridation à une région située sur le gène de CK20 en aval de la troisième amorce ;
et
dans lequel lesdites sondes pour l'ARNm de CK20 sont
- une troisième sonde ayant une séquence permettant son hybridation à une région située sur le gène de CK20 en aval de la région à laquelle ladite troisième amorce se lie et en amont de la région à laquelle ladite quatrième amorce se lie, et
- une quatrième sonde ayant une séquence permettant son hybridation à une région située sur le gène de CK20 en amont de la région à laquelle ladite quatrième amorce se lie et en aval de la région à laquelle ladite troisième sonde se lie ;
dans lequel les sondes pour la détection de CK19 sont marquées avec un donneur et un accepteur et les sondes pour la détection de CK20 sont marquées avec un donneur et un accepteur, et dans lequel la paire de donneur/accepteur pour CK19 est différente de la paire de donneur/accepteur pour CK20.

5. Procédé selon la revendication 4, dans lequel le jeu de sondes pour la détection de CK19 et le jeu de sondes pour la détection de CK20 contiennent le même donneur, tandis que l'accepteur pour la détection de CK19 est différent de l'accepteur pour la détection de CK20.

6. Procédé selon les revendications 4 ou 5, dans lequel la première et la troisième sonde sont marquées avec un donneur à leur extrémité 3', et dans lequel la deuxième et la quatrième sonde sont marquées avec un accepteur à leur extrémité 5'.

7. Procédé selon l'une quelconque des revendications 4 à 6, dans lequel le donneur est le FITC et l'accepteur est choisi dans un groupe constitué par les colorants de type rhodamine et les colorants de type cyanine.

8. Trousse pour l'amplification et la détection de l'ARNm de CK19, dans laquelle ladite trousse comprend au moins
une paire d'amorces avec
- une première amorce comprenant SEQ ID NO: 2, et
- une deuxième amorce comprenant SEQ ID NO: 4 ;
ainsi qu'une paire de sondes avec
- une première sonde s'hybridant à une région située à l'extrémité 3' d'un exon du gène de CK19 en aval de la région à laquelle ladite première amorce se lie et en amont de la région à laquelle ladite deuxième amorce se lie, et
- une deuxième sonde s'hybridant à une région située à l'extrémité 5' d'un exon adjacent au gène de CK19 en aval de la région à laquelle ladite première sonde se lie et en amont de la région à laquelle ladite deuxième amorce se lie ;
l'une desdites sondes étant marquée avec un donneur et l'autre desdites sondes étant marquée avec un accepteur.

9. Trousse selon la revendication 8 pour l'amplification et la détection combinées de l'ARNm de CK19 et l'ARNm de CK20, comprenant en outre au moins une deuxième paire d'amorces et une deuxième paire de sondes, les sondes pour la détection de CK19 étant marquées avec un donneur et un accepteur et les sondes pour la détection de CK20 étant marquées avec un donneur et un accepteur, dans laquelle la paire de donneur/accepteur pour CK19 est différente de la paire de donneur/accepteur pour CK20.

10. Trousse selon la revendication 9, dans laquelle lesdites amorces pour l'ARNm de CK20 sont
- une troisième amorce ayant une séquence permettant son hybridation à une région située sur le gène de CK20, et
- une quatrième amorce ayant une séquence permettant son hybridation à une région située sur le gène de CK20 en aval de la troisième amorce ; et
dans laquelle lesdites sondes pour l'ARNm de CK20 sont
- une troisième sonde ayant une séquence permettant son hybridation à une région située sur le gène de CK20 en aval de la région à laquelle ladite troisième amorce se lie et en amont de la région à laquelle ladite quatrième amorce se lie, et
- une quatrième sonde ayant une séquence permettant son hybridation à une région située sur le gène de CK20 en amont de la région à laquelle ladite quatrième amorce se lie et en aval de la région à laquelle ladite troisième sonde se lie.

11. Trousse selon les revendications 8 à 10, dans laquelle les paires d'amorces sont contenues dans un mélange ou dans laquelle les paires de sondes sont contenues dans un mélange ou dans laquelle les paires d'amorces et les paires de sondes sont contenues dans un mélange.

12. Trousse selon l'une quelconque des revendications 8 à 11, contenant en outre une solution tampon.


### SEQUENCE LISTING

<110>F. Hoffmann-La Roche AG
<120>Method for Quick Determination of Cytokeratin 19 (CK19) and Primers and
   Probes therefore
<130>b056769
< 140>EP07007289.7
   <141>2007-04-07
<160>16
<210>1
   <211>115
   <212>DNA
   <213>Homo sapiens
<220>
   <223>Part of mRNA encoding cytokeratin 19
<400>1
   ggtggattcc gctccgggca ccgatctcgc caagatcctg agtgacatgc gaagccaata 60
   tgaggtcatg gccgagcaga accggaagga tgctgaagcc tggttcacca gccgg 115
<210>2
   <211>17
   <212>DNA
   <213>Artificial Sequence
<220>
   <223>Primer F1
<400>2
   tgagtgacat gcgaagc 17
<210>3
   <211>559
   <212>DNA
   <213>Homo sapiens
<220>
   <223>Part of mRNA encoding cytokeratin 19
<400>3
   gatgctgaag cctggttcac cagccggact gaagaattga accgggaggt cgctggccac 60
   acggagcagc tccagatgag caggtccgag gttactgacc tgcggcgcac ccttcagggt 120
   cttgagattg agctgcagtc acagctgagc atgaaagctg ccttggaaga cacactggca 180
   gaaacggagg cgcgctttgg agcccagctg gcgcatatcc aggcgctgat cagcggtatt 240
   gaagcccagc tgggcgatgt gcgagctgat agtgagcggc agaatcagga gtaccagcgg 300
   ctcatggaca tcaagtcgcg gctggagcag gagattgcca cctaccgcag cctgctcgag 360
   ggacaggaag atcactacaa caatttgtct gcctccaagg tcctctgagg cagcaggctc 420
   tggggcttct gctgtccttt ggagggtgtc ttctgggtag agggatggga aggaagggac 480
   ccttaccccc ggctcttctc ctgacctgcc aataaaaatt tatggtccaa gggaaaaaaa 540
   aaaaaaaaaa aaaaaaaaa 559
<210>4
   <211>16
   <212>DNA
   <213>Artificial Sequence
<220>
   <223>Primer R1
<400>4
   tgtgtcttcc aaggca 16
<210>5
   <211>1407
   <212>DNA
   <213>Homo apiens
<220>
   <221>exon
   <222>1..473
<220>
   <221>exon
   <222>374..556
<220>
   <221>exon
   <222>557..713
<220>
   <221>exon
   <222>714..875
<220>
   <221>exon
   <222>876..1001
<220>
   <221>exon
   <222>1002..1380
<400>5
   cgcgaatcgc agcttctgag accagggttg ctccgtccgt gctccgcctc gccatgactt 60
   cctacagcta tcgccagtcg tcggccacgt cgtccttcgg aggcctgggc ggcggctccg 120
   tgcgttttgg gccgggggtc gcctttcgcg cgcccagcat tcacgggggc tccggcggcc 180
   gcggcgtatc cgtgtcctcc gcccgctttg tgtcctcgtc ctcctcgggg gcctacggcg 240
   gcggctacgg cggcgtcctg accgcgtccg acgggctgct ggcgggcaac gagaagctaa 300
   ccatgcagaa cctcaacgac cgcctggcct cctacctgga caaggtgcgc gccctggagg 360
   cggccaacgg cgagctagag gtgaagatcc gcgactggta ccagaagcag gggcctgggc 420
   cctcccgcga ctacagccac tactacacga ccatccagga cctgcgggac aagattcttg 480
   gtgccaccat tgagaactcc aggattgtcc tgcagatcga caatgcccgt ctggctgcag 540
   atgacttccg aaccaagttt gagacggaac aggctctgcg catgagcgtg gaggccgaca 600
   tcaacggcct gcgcagggtg ctggatgagc tgaccctggc caggaccgac ctggagatgc 660
   agatcgaagg cctgaaggaa gagctggcct acctgaagaa gaaccatgag gaggaaatca 720
   gtacgctgag ggccaagtgg ggaggccagg tcagtgtgga ggtggattcc gctccgggca 780
   ccgatctcgc caagatcctg agtgacatgc gaagccaata tgaggtcatg gccgagcaga 840
   accggaagga tgctgaagcc tggttcacca gccggactga agaattgaac cgggaggtcg 900
   ctggccacac ggagcagctc cagatgagca ggtccgaggt tactgacctg cggcgcaccc 960
   ttcagggtct tgagattgag ctgcagtcac agctgagcat gaaagctgcc ttggaagaca 1020
   cactggcaga aacggaggcg cgctttggag cccagctggc gcatatccag gcgctgatca 1080
   gcggtattga agcccagctg ggcgatgtgc gagctgatag tgagcggcag aatcaggagt 1140
   accagcggct catggacatc aagtcgcggc tggagcagga gattgccacc taccgcagcc 1200
   tgctcgaggg acaggaagat cactacaaca atttgtctgc ctccaaggtc ctctgaggca 1260
   gcaggctctg gggcttctgc tgtcctttgg agggtgtctt ctgggtagag ggatgggaag 1320
   gaagggaccc ttacccccgg ctcttctcct gacctgccaa taaaaattta tggtccaagg 1380
   gaaaaaaaaa aaaaaaaaaa aaaaaaa 1407
<210>6
   <211>19
   <212>DNA
   <213>Artificial Sequence
<220>
   <223>Probe P1b
<400>6
   aagcctggtt caccagccg 19
<210>7
   <211>21
   <212>DNA
   <213>Artificial Sequence
<220>
   <223>Probe P2c
<400>7
   ctgaagaatt gaaccgggag g 21
<210>8
   <211>17
   <212>DNA
   <213>Artificial Sequence
<220>
   <223>Primer F2
<400>8
   cgccaagatc ctgagtg 17
<210>9
   <211>18
   <212>DNA
   <213>Artificial Sequence
<220>
   <223>Primer F3
<400>9
   gacatgcgaa gccaatat 18
<210>10
   <211>16
   <212>DNA
   <213>Artificial Sequence
<220>
   <223>Primer R2
<400>10
   ccaaggcagc tttcat 16
<210>11
   <211>19
   <212>DNA
   <213>Artificial Sequence
<220>
   <223>Probe P1
<400>11
   gtcatggccg agcagaacc 19
<210>12
   <211>19
   <212>DNA
   <213>Artificial Sequence
<220>
   <223>Primer F1
<400>12
   aaggatgctg aagcctggt 19
<210>13
   <211>20
   <212>DNA
   <213>Artificial Sequence
<220>
   <223>Primer CK20F
<400>13
   atcaagcagt ggtacgaaac 20
<210>14
   <211>18
   <212>DNA
   <213>Artificial Sequence
<220>
   <223>Primer CK20R
<400>14
   aggacacacc gagcattt 18
<210>15
   <211>25
   <212>DNA
   <213>Artificial Sequence
<220>
   <223>Probe CK20P1
<400>15
   attacagaca aattgaagag ctgcc 25
<210>16
   <211>25
   <212>DNA
   <213>Artificial Sequence
<220>
   <223>Primer CK20P2
<400>16
   agtcagatta aggatgctca actgc 25